# EUROPEAN PATENT APPLICATION

(11) **EP 2 399 564 A1**
(43) Date of publication of application: **28.12.2011**
(21) Application number: 10167514.8
(22) Date of filing: 28.06.2010
(51) Int. Cl.: A61J 1/20, A61M 5/24, B65D 81/32

(54) **Fluid separation device for fluid storage and mixing apparatus, and fluid storage and mixing apparatus incorporating such device**

(71) Applicant: THE UNIVERSITY OF NEWCASTLE UPON TYNE, Newcastle Upon Tyne and Wear NE1 7RU (GB)
(72) Inventor: Meechan, John gerard, Northumberland, NE43 7 TW (GB); Lawrence, Christopher David, Northumberland, NE43 7SX (GB); Harley, Phillip Edward, Northumberland, NE47 6NF (GB)
(74) Representative: Vinsome, Rex Martin

(57) **Abstract**

A fluid separation device for a cartridge (2) for dental anaesthetic is disclosed. The device comprises a first piston (10) adapted to sealingly engage an internal wall of a body (8) of the cartridge to define a boundary of a first fluid chamber (16) and to slide relative to the body when a force greater than a predetermined first level is applied thereto, and a second piston (14) adapted to sealingly engage an internal wall of the cartridge body to define a boundary between the first fluid chamber and a second fluid chamber (20) and to allow fluid to flow from the first fluid chamber to the second fluid chamber when a force greater than a predetermined second level, greater than the first predetermined level, is applied to the second member.

## Description

The present invention relates to a fluid separation device for a fluid storage and mixing apparatus, and relates particularly, but not exclusively, to fluid separation device for a dental local anaesthetic cartridge.

It has been found that acidity of local anaesthetic media contributes to pain experienced by a patient receiving a dental local anaesthetic injection. The acidity is necessary to preserve the efficacy of one of the components of the solution (adrenaline) during storage. As a result, an acidity regulator can be added immediately prior to use, and the discomfort caused by the injection can be moderated with minimal effect on the efficacy of the anaesthetic.

US5603695 discloses a disposable cartridge containing a buffering solution for alkalising local anaesthetic injection medication. The cartridge is provided with a needle to enable the buffering solution to be added to the anaesthetic fluid immediately before use.

This arrangement suffers from the drawback that in addition to containers of local anaesthetic, disposable containers of buffering solution must be obtained and disposed of after use. This significantly increases the inconvenience and cost involved. In addition, the use of a two-part assembly increases the inconvenience to the user.

Preferred embodiments of the present invention seek to overcome one or more of the above disadvantages of the prior art.

According to an aspect of the present invention, there is provided a fluid separation device for a fluid storage and mixing apparatus having a container body for containing first and second fluids, the fluid separation device comprising:-
a first member adapted to sealingly engage an internal wall of the container body to define a boundary of a first fluid chamber and to slide relative to said container body when a force greater than a predetermined first level is applied thereto;
a second member adapted to sealingly engage an internal wall of the container body to define a boundary between said first fluid chamber and a second fluid chamber and to allow fluid to flow from said first fluid chamber to said second fluid chamber when a force greater than a predetermined second level, greater than said first predetermined level, is applied to said second member.

By providing a second member adapted to sealingly engage an internal wall of the container body to define a boundary between the first fluid chamber and a second fluid chamber and to allow fluid to flow from the first fluid chamber to the second fluid chamber when a force greater than a predetermined second level is applied to the second member, this provides the advantage of enabling mixing of first and second fluids by means of a single component, thereby increasing the ease of use of the fluid storage and mixing apparatus.

The second member may be adapted to allow fluid to flow from said first fluid chamber to said second fluid chamber by means of at least one insert, adapted to be releasably mounted to a respective channel through said second member.

This provides the advantage of enabling the insert, when released from the channel, to contribute to mixing of the two fluids by being moveable throughout the second fluid chamber.

The second member may be adapted to allow fluid to flow from said first fluid chamber to said second fluid chamber by means of valve means provided on said second member.

The second member may be adapted to allow fluid to flow from said first fluid chamber to said second fluid chamber by means of at least one rupturable membrane provided on said second member.

The second member may be adapted to slide relative to said container body when a force greater than a predetermined third level, greater than said predetermined second level, is applied to said second member.

This provides the advantage of enabling the second member to contribute to drug delivery, for example by enabling a syringe actuator to displace both the first and second member.

The device may comprise at least one respective sealing element for sealing said first member and said second member to said body.

According to another aspect of the present invention, there is provided a fluid storage and mixing apparatus comprising a container body and a fluid separation device as defined above mounted to said container body.

The apparatus may further comprise acidity regulator in said first fluid chamber and anaesthetic fluid in said second fluid chamber.

The apparatus may further comprise acidified (adrenaline-containing) solution in said first fluid chamber and anaesthetic fluid in said second fluid chamber.

The acidified solution may contain adrenaline.

A preferred embodiment of the invention will now be described, by way of example only and not in any limitative sense, with reference to the accompanying drawings, in which:-
Figure 1 is a schematic cross-sectional view of a fluid storage and mixing apparatus embodying the present invention;
Figure 2 is a cross-sectional view of part of the apparatus of Figure 1; and
Figures 3 to 5 show a fluid mixing process of the part of the apparatus shown in Figure 2.

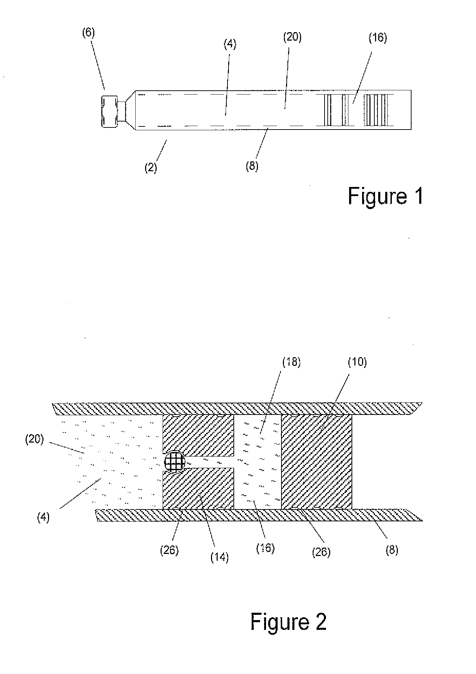

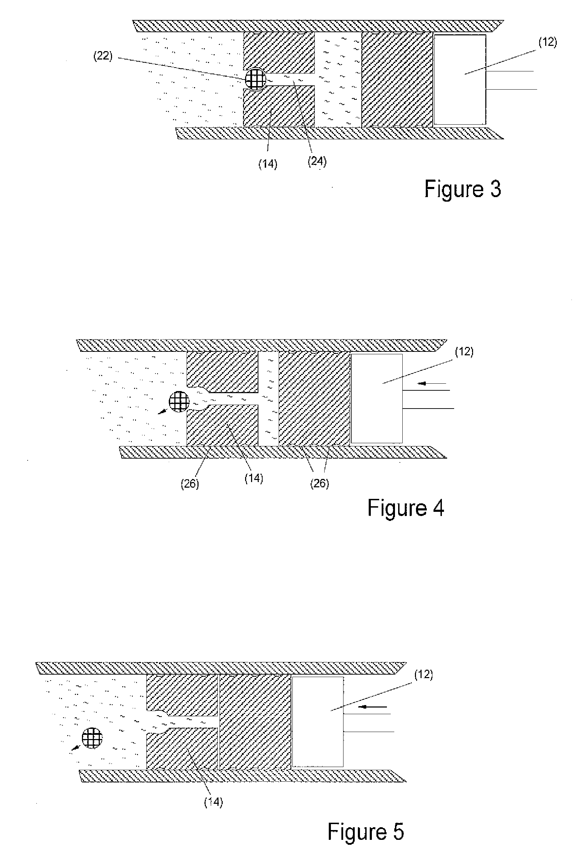

Referring to Figure 1, a fluid storage and mixing apparatus in the form of a cartridge 2 for dental anaesthetic 4 for use with a syringe (not shown) includes a cap 6 containing a seal adapted to be penetrated by a needle incorporated into a syringe (not shown) into which the cartridge 2 is inserted prior to clinical use. The cartridge 2 is provided with a hollow cylindrical body 8 in which a first member in the form of a first piston 10 is slidably mounted and is engageable by a syringe activator 12 (Figures 3 to 5) pressed by a clinician's thumb to deliver anaesthetic fluid 4 through a syringe needle (not shown) to a patient.

A second body in the form of a second piston 14 is slidably mounted to the body 8 to separate a first fluid chamber 16 containing an acidity regulator 18 (or adrenaline-containing fraction) from a second fluid chamber 20 containing the anaesthetic 4. The second piston 14 is provided with a releasable separator in the form of a ball 22 releasably mounted in a channel 24. The frictional resistance between the second piston 14 and the wall of the body 8 is greater than that between the first piston 10 and the body 8. The first and second pistons sealingly engage the body 8 by means of seals 26.

The operation of the device will now be described with reference to Figures 3 to 5.

When the syringe actuator 12 is initially actuated by being depressed by the clinician's thumb, the pressure on the acidity regulator 18 (or adrenaline-containing fraction)in the first fluid chamber 16 increases until the ball 22 is ejected from the channel 24 through the second piston 14, as shown in Figure 4. This allows the first piston 10 to move towards the second piston 14, as a result of which the acidity regulator 18 in the first fluid chamber 16 is expelled through the channel 24 and mixed with the anaesthetic 4 in the second chamber 20, the released ball 22 contributing to the mixing of the two fluids. When the first piston 10 comes into contact with the second piston 14, application of further force causes both pistons to slide relative to the body 8 to eject a mixture of anaesthetic 4 and acidity regulator 18 (or adrenaline-containing fraction)through the needle (not shown) of the syringe.

It will be appreciated by persons skilled in the art that the above embodiment has been described by way of example only, and not in any limitative sense, and that various alterations and modifications are possible without departure from the scope of the invention as defined by the appended claims.

## Claims

1. A fluid separation device for a fluid storage and mixing apparatus having a container body for containing first and second fluids, the fluid separation device comprising:-
a first member adapted to sealingly engage an internal wall of the container body to define a boundary of a first fluid chamber and to slide relative to said container body when a force greater than a predetermined first level is applied thereto;
a second member adapted to sealingly engage an internal wall of the container body to define a boundary between said first fluid chamber and a second fluid chamber and to allow fluid to flow from said first fluid chamber to said second fluid chamber when a force greater than a predetermined second level, greater than said first predetermined level, is applied to said second member.

2. A device according to claim 1, wherein said second member is adapted to allow fluid to flow from said first fluid chamber to said second fluid chamber by means of at least one insert, adapted to be releasably mounted to a respective channel through said second member.

3. A device according to claim 1 or 2, wherein said second member is adapted to allow fluid to flow from said first fluid chamber to said second fluid chamber by means of valve means provided on said second member.

4. A device according to any one of the preceding claims, wherein said second member is adapted to allow fluid to flow from said first fluid chamber to said second fluid chamber by means of at least one rupturable membrane provided on said second member.

5. A device according to any one of the preceding claims, wherein said second member is adapted to slide relative to said container body when a force greater than a predetermined third level, greater than said predetermined second level, is applied to said second member.

6. A device according to any one of the preceding claims, further comprising at least one respective sealing element for sealing said first member and said second member to said body.

7. A fluid storage and mixing apparatus comprising a container body and a fluid separation device according to any one of the preceding claims mounted to said container body.

8. An apparatus according to claim 7, further comprising acidity regulator in said first fluid chamber and anaesthetic fluid in said second fluid chamber.

9. An apparatus according to claim 7, further comprising acidified solution in said first fluid chamber and anaesthetic fluid in said second fluid chamber.

10. An apparatus according to claim 9, wherein the acidified solution contains adrenaline.
